Europäisches Patentamt

European Patent Office

Office européen des brevets

(19)

(11) Numéro de publication: **0 354 096**

**A1**

# (12) DEMANDE DE BREVET EUROPEEN

(21) Numéro de dépôt: **89402114.6**

(22) Date de dépôt: **25.07.89**

(51) Int. Cl.5: **G 01 N 17/00**

(30) Priorité: **26.07.88 FR 8810064**

(43) Date de publication de la demande:
**07.02.90 Bulletin 90/06**

(84) Etats contractants désignés:
**AT BE CH DE ES FR GB GR IT LI LU NL SE**

(71) Demandeur: **ETAT FRANCAIS, représenté par le MINISTERE DE L'EQUIPEMENT, DU LOGEMENT, DE L'AMENAGEMENT DU TERRITOIRE ET DES TRANSPORTS, LAB ORATOIRE CENTRAL DES PONTS ET CHAUSSESS 58, boulevard Lefèbre, F-75015 Paris (FR)**

**ADVANCED TECHNOLOGIES INDUSTRY 6, rue Jean-Pierre Timbaud F-78180 Montigny le Bretonneux (FR)**

(72) Inventeur: **Raharinaivo, André 378, rue de Vaugirard F-75015 Paris (FR)**

**Grimaldi, Gilbert 5, rue Grande F-77820 Les Ecrennes (FR)**

**Fontaine, Guy 24, rue de Passy F-75016 Paris (FR)**

**Delsol, Franck 36, Grande rue La Roseraie Bâtiment B F-91520 Egly (FR)**

(74) Mandataire: **Nony, Michel et al Cabinet NONY & CIE 29, rue Cambacérès F-75008 Paris (FR)**

(54) **Procédé de diagnostic de la corrosion des aciers dans les bétons.**

(57) L'invention est relative à un procédé de diagnostic de la corrosion des ferraillages d'une structure en béton armé dans lequel on applique au moins un signal électrique entre ledit ferraillage et une électrode placée à la surface du béton au voisinage dudit ferraillage.

On relève les caractéristiques de la tension et de l'intensité dudit signal et on déduit desdites caractéristiques au moins un paramètre représentatif de l'état de corrosion du ferraillage.

Fig.1

EP 0 354 096 A1

**Description**

## Procédé de diagnostic de la corrosion des aciers dans les bétons.

La présente invention concerne un procédé de diagnostic de la corrosion des ferraillages d'une structure en béton armé.

Le béton armé est composé, outre son ferraillage, d'un certain nombre d'éléments : ciment, agrégats, eau, ainsi qu'éventuellement des adjuvants.

Après durcissement, le béton armé présente, notamment en ce qui concerne les potentialités de corrosion, les propriétés physiques et électriques suivantes :
- le béton doit être considéré, sauf au niveau des surfaces externes par temps sec, comme un matériau présentant une certaine conductibilité électrique. L'eau n'est effectivement pas utilisée intégralement dans les réactions chimiques initiales ;
- le béton est naturellement basique et présente généralement un pH de l'ordre de 12,5 à 13. Ce phénomène est dû à la libération de chaux dans le béton.

Cette dernière remarque entraîne en conséquence une passivation absolue du ferraillage dans le béton à la condition toutefois que cette alcalinité ne soit partiellement ou totalement neutralisée par la présence d'ions $Cl^-$ et de $CO_2$.

La présence d'ions $Cl^-$ exerce un double effet, à savoir, la suppression de la passivation de l'acier par réduction du pH, et l'accroissement de la conductibilité du béton.

Par ailleurs, la présence de gaz carbonique liée généralement à la pollution ambiante, peut entraîner, sous la forme d'acide carbonique, la saturation des composés alcalins du ciment, d'où une baisse notable du pH et une disparition de la passivation des armatures; c'est ce phénomène qui est connu sous le nom de carbonatation.

Enfin, certains éléments chimiques peuvent augmenter la porosité et la perméabilité du béton et accroître ainsi considérablement les risques de corrosion des ferraillages.

La corrosion des structures en béton armé entraîne à plus ou moins long terme de nombreuses défaillances dues à l'affaiblissement de leur résistance mécanique et à leur perte de fiabilité, ces défaillances pouvant avoir des conséquences sérieuses telles que des réductions des normes d'exploitation, ou même catastrophiques comme l'effondrement des structures.

Il est donc essentiel de pouvoir effectuer chaque fois que cela se révèle nécessaire un diagnostic précis de la situation de corrosion en chaque point de la structure concernée.

A cet effet, l'invention a pour objet un procédé de diagnostic de la corrosion des ferraillages d'une structure en béton armé, caractérisé par le fait que l'on applique au moins un signal électrique entre ledit ferraillage et au moins une électrode placée à la surface du béton au voisinage dudit ferraillage, que l'on relève les caractéristiques de la tension et de l'intensité dudit signal, et que l'on déduit desdites caractéristiques au moins un paramètre représentatif de l'état de corrosion du ferraillage.

Dans un premier mode de réalisation de l'invention, l'un au moins desdits signaux électriques est un signal alternatif de fréquence prédéterminée et notamment, dans des gammes spécifiques de fréquence. On détermine l'angle de déphasage entre son intensité et sa tension et l'on déduit l'état de corrosion du ferraillage dudit angle de déphasage.

On verra ci-après que, dans le cas où le métal du ferraillage est passivé, le comportement de l'ensemble ferraillage-béton est essentiellement capacitif, l'angle de déphasage entre l'intensité et la tension étant alors plus près de 90°. Au contraire, en situation de corrosion, le comportement de la structure est essentiellement galvanique, l'angle de déphasage étant alors plus près de 0°.

Dans un deuxième mode de réalisation de l'invention, l'un au moins desdits signaux est un signal alternatif dans une gamme de fréquences relativement basse, et l'on déduit la résistance de polarisation du ferraillage du rapport entre les amplitudes de sa tension et de son intensité.

Plus particulièrement, on peut appliquer deux signaux alternatifs, l'un dans une gamme de fréquences relativement basse et l'autre dans une gamme de fréquences relativement haute, et déduire en outre la résistance du béton compris entre le ferraillage et l'électrode, du rapport entre les amplitudes de la tension et de l'intensité du deuxième signal alternatif.

On verra en effet que, pour une fréquence relativement basse, par exemple pour une fréquence comprise entre 1 Hz et 10 KHz et plus particulièrement entre 10 Hz et 1 KHz le rapport tension/courant est avant tout significatif de la résistance de polarisation du ferraillage, alors que pour une fréquence relativement haute, par exemple pour une fréquence comprise entre 50 KHz et quelques MHz et, plus particulièrement, entre 100 KHz et 1 MHz, ce rapport est essentiellement significatif de la résistance du béton.

Selon un troisième mode de réalisation de l'invention, on applique au moins deux signaux électriques, et l'on déduit la résistance de polarisation du ferraillage du gradient amplitude de l'intensité/amplitude de la tension.

On verra en effet que la tangente de l'angle $\Delta i/\Delta v$ caractérise la polarisation du ferraillage.

Dans le cas où l'on applique successivement deux signaux électriques continus, ou peut également déduire la résistance de polarisation de l'analyse du courant transitoire lors de la phase de commutation.

On peut compléter les résultats obtenus ci-dessus en interrompant le signal électrique appliqué, et en mesurant alors, d'une part la tension du ferraillage par rapport à la surface du béton, et d'autre part la tension du ferraillage par rapport à son environnement direct, et en déduisant l'état de corrosion du ferraillage du signe de la différence entre ces deux tensions.

En effet, lorsqu'un courant électrique entre dans le ferraillage ou en sort, des lignes de potentiel apparaissent dans le béton. Si ce courant entre dans le ferraillage, ces potentiels deviennent de plus en plus négatifs en s'éloignant du ferraillage, le comportement de ce dernier est alors cathodique et il ne se corrode pas. Si au contraire un courant électrique sort du ferraillage, les potentiels sont de moins en moins négatifs en s'en éloignant, de sorte que son comportement est anodique et qu'il y a un risque de corrosion.

Bien entendu deux ou plusieurs des modes de réalisation décrits ci-dessus peuvent être mis en oeuvre, soit séquentiellement, soit simultanément à l'aide de filtres, afin d'obtenir une meilleure précision.

On remarquera en effet que le premier mode de réalisation donne des résultats essentiellement qualitatifs puisqu'il permet de dire si l'on est en situation d'absence ou de présence de corrosion selon que l'angle de phase entre l'intensité et la tension est plus près de 90° ou de 0° respectivement. Au contraire, les deuxième et troisième modes de réalisation donnent des résultats quantitatifs puisqu'ils permettent d'obtenir soit la résistance de polarisation du ferraillage, soit la densité du courant sortant.

Les mesures peuvent également être répétées cycliquement le long du ferraillage, par exemple en utilisant des roues, des frotteurs ou tout autre système de contact mobile permanent comme électrodes.

On décrira maintenant divers modes de réalisation de l'invention en référence aux dessins schématiques annexés dans lesquels :

- La figure 1 illustre le principe des mesures effectuées dans le cadre de la mise en oeuvre du procédé selon l'invention,

- les figures 2 et 3 illustrent deux variantes d'un mode de réalisation de l'invention,

- la figure 4 correspond à la figure 3 en fonctionnement multi-voies,

- les figures 5 et 6 sont deux schémas équivalents d'une portion de béton armé,

- la figure 7 illustre un autre mode de réalisation de l'invention et,

- les figures 8 et 9 illustrent un procédé complémentaire susceptible d'être mis en oeuvre dans le cadre de l'invention.

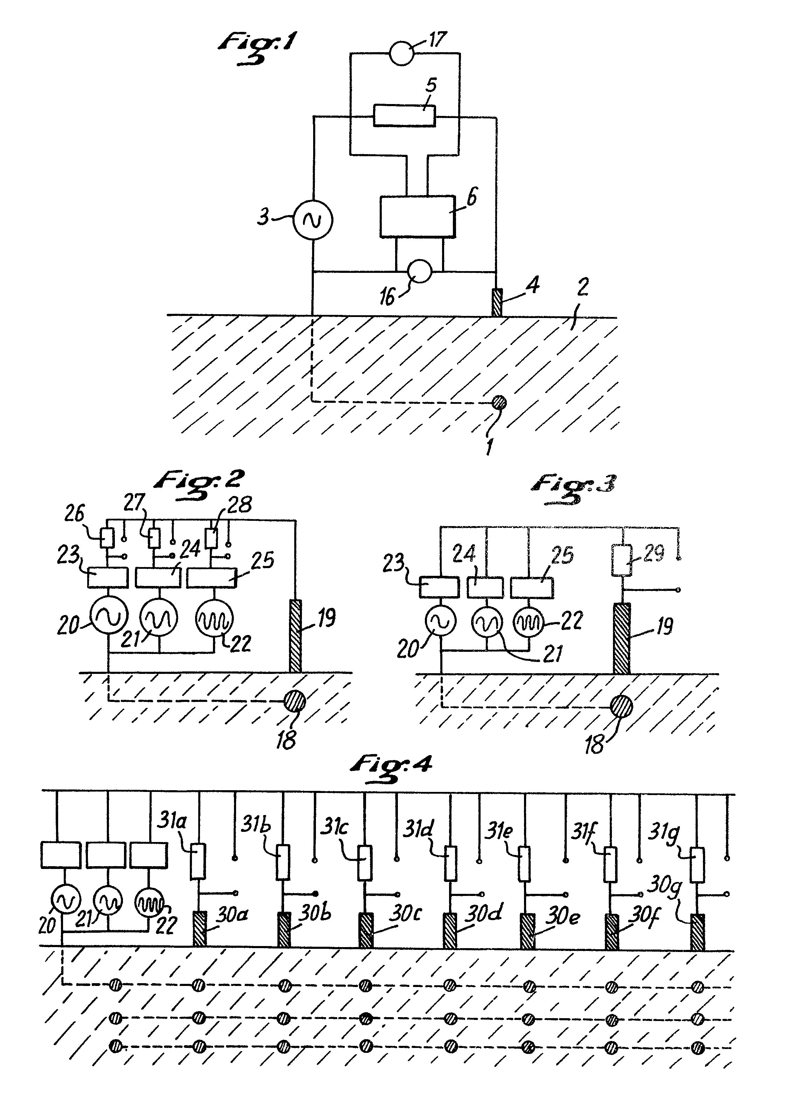

La figure 1 représente une portion de béton armé dans laquelle un ferraillage 1 est noyé dans du béton 2.

L'invention consiste à déterminer l'état de corrosion du ferraillage 1 en appliquant un signal électrique à l'aide d'un générateur 3 entre le ferraillage 1 et une électrode métallique 4 placée à la surface du béton au voisinage du ferraillage.

Une résistance 5 est mise en série avec le générateur 3 pour caractériser le courant dans le circuit par la tension à ses bornes.

Un premier mode de réalisation de l'invention consiste à mesurer le déphasage entre l'intensité et la tension du signal appliqué entre le ferraillage et la surface du béton. A cet effet, un phasemètre 6 est connecté d'une part entre les bornes de la résistance 5 et d'autre part, entre le ferraillage 1 et l'électrode 4.

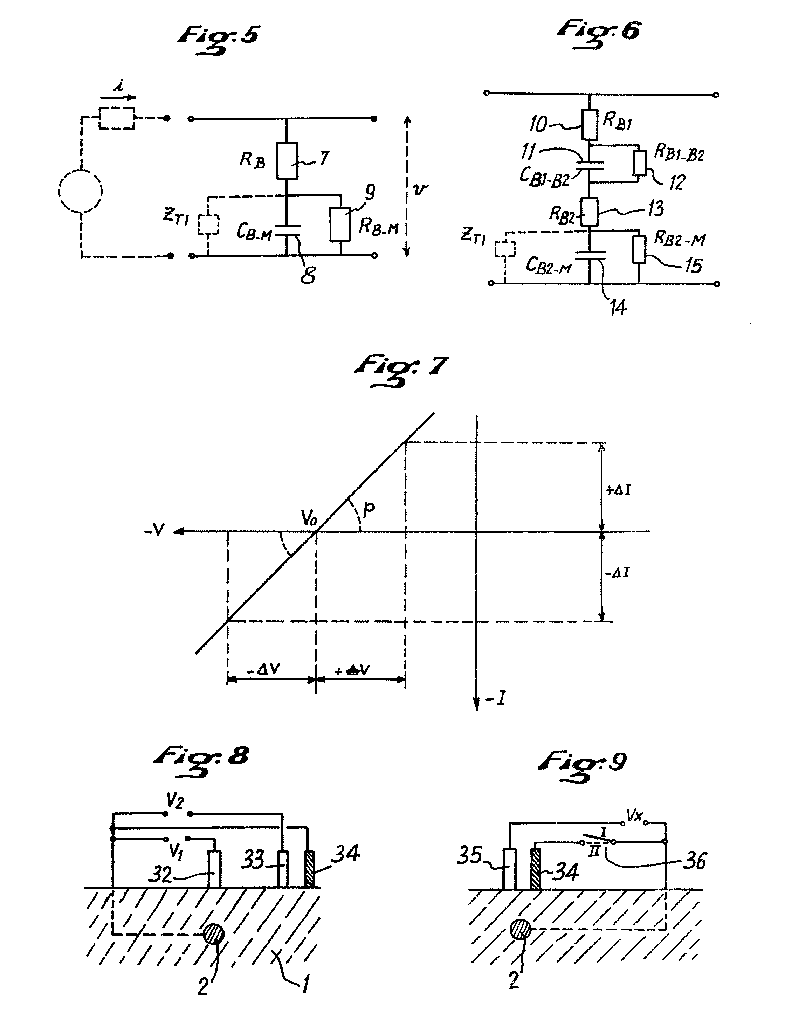

Si l'on se refère en effet à la figure 5, on constate que le schéma équivalent de l'ensemble constitué par le béton et le ferraillage et constitué par la résistance 7 du béton $R_B$ en série avec l'impédance de l'interface béton/métal elle-même constituée par une capacité d'interface 8 $C_{BM}$ en parallèle avec une résistance 9 $R_{BM}$, ainsi qu'une impédance interfacielle de transfert $Z_{TI}$.

Si l'on applique aux bornes de ce circuit une tension alternative v, il en résulte un courant i.

Pour une fréquence relativement basse, par exemple comprise entre 1Hz et 10 KHz, l'impédance de la branche capacitive est prépondérante de sorte que le rapport tension v/courant i est essentiellement représentatif de la résistance interfacielle $R_{BM}$, c'est-à-dire de la résistance de polarisation du ferraillage.

Au contraire, pour une fréquence plus élevée, par exemple comprise entre 50KHz et quelques MHz et de préférence entre 100 KHz et 1MHz, l'impédance de la branche capacitive diminue fortement et la résistance du béton $R_B$ devient l'élément prépondérant du fait du shuntage très important de la résistance $R_B$.

Par conséquent, en situation normale, c'est-à-dire lorsque le métal du ferraillage est passivé, le comportement du circuit équivalent aux basses fréquences est principalement capacitif, de sorte que l'angle de déphasage entre la tension et l'intensité se rapproche de 90°.

Au contraire, si le ferraillage est en état de corrosion, la résistance interfacielle $R_{BM}$ a considérablement diminué de sorte que, pour les mêmes fréquences, le comportement électrique est essentiellement galvanique, de sorte que l'angle de déphasage se rapproche de 0°.

Il est donc possible en mesurant le déphasage à relativement basse fréquence d'en déduire une appréciation au moins qualitative de l'état de corrosion du ferraillage.

Par contre, aux fréquences plus élevées, la résistance de l'ensemble est essentiellement caractéristique de la résistance du béton.

La figure 6 illustre le cas plus complexe où une couche isolante, par exemple, une membrane de matière plastique, est noyée dans le béton entre le ferraillage et la surface afin de protéger ce ferraillage de l'humidité ambiante.

Dans ce cas, la résistance $R_{B1}$ de la couche de béton de surface est représentée en 10.

La capacité $C_{B1B2}$ et la résistance $R_{B1B2}$ de la couche isolante sont représentées respectivement en 11 et 12.

La résistance $R_{B2}$ de la deuxième couche de béton est représentée en 13.

La capacité $C_{B2M}$ et la résistance $R_{B2M}$ de l'interface béton/ferraillage sont représentées respectivement en 14 et 15, ainsi que l'impédance interfacielle de transfert $Z_{TI}$.

Dans le cas normal où la couche isolante est en bon état et où le métal du ferraillage est passivé, les capacités 11 et 14 sont prépondérantes, de sorte que le comportement de l'ensemble est essentiellement capacitif pour toutes les fréquences.

Dans ce cas, le déphasage entre l'intensité et la tension appliquée se rapproche de 90°.

Si la couche isolante est dégradée, sa capacité devient négligeable, de sorte que l'on est ramené au cas de la figure 5 et que le comportement de l'ensemble n'est capacitif qu'à basse fréquence, dans le cas où le ferraillage est passivé.

Enfin, dans le cas où à la fois la couche isolante est dégradée et le métal du ferraillage est en état de corrosion, le comportement est purement résistif de sorte que le déphasage tend vers 0.

On constate par conséquent qu'il est non seulement possible d'obtenir une estimation qualitative de l'état de corrosion du ferraillage à l'aide de l'angle de déphasage entre l'intensité et la tension, mais également d'obtenir une valeur quantitative de la résistance de polarisation du ferraillage en faisant le rapport de la tension appliquée à l'intensité qui parcourt le circuit.

Si l'on revient à la figure 1, ce rapport est calculé à un coefficient près à partir des indications données, pour la tension par un dispositif 16 connecté entre l'électrode 4 et le ferraillage 1, et pour l'intensité par une chaîne d'acquisition 17 connectée aux bornes de la résistance 5, et par application de la loi d'Ohm $(i = v/z)$

Enfin, les dispositifs de mesure de tension 16 et 17 permettent la mise en oeuvre d'un troisième mode de réalisation de l'invention.

Si l'on applique en effet, comme montré à la figure 7, deux signaux électriques continus ou alternatifs entre l'électrode 4 et le ferraillage 1, il est possible d'obtenir directement la résistance de polarisation.

La figure 7 représente deux valeurs de la tension situées de part et d'autre d'une valeur médiane $V_0$, à savoir $V_0 - \Delta v$ et $V_0 + \Delta v$. Le courant qui en résulte est respectivement $-\Delta i$ et $+\Delta i$.

On peut montrer que la tangente de l'angle $\rho$, c'est-à-dire la pente de la caractéristique tension/intensité, est inversement proportionnelle à la résistance de polarisation du ferraillage. Les différentes mesures ci-dessus peuvent être effectuées simultanément ou successivement.

Les figures 2 ou 3 représentent le cas où trois signaux alternatifs sont appliqués simultanément entre un ferraillage 18 et une électrode 19 placée à la surface du béton.

Dans ce cas, trois générateurs 20, 21, 22 sont connectés en parallèle et munis de filtres passe-bande 23, 24 et 25 respectivement.

Dans le cas de la figure 2, chaque ensemble générateur-filtre est disposé en série avec une résistance 26, 27, 28 respectivement. La tension entre le ferraillage et l'électrode est mesurée comme précédemment dans la figure 1, et l'intensité du courant à chaque fréquence est caractérisée par la tension aux bornes des résistances 26, 27 et 28.

On peut encore obtenir à chacune de ces fréquences le rapport tension/intensité et ainsi en déduire la résistance de polarisation du ferraillage et la résistance du béton, sous réserve que les fréquences des générateurs soient convenablement réparties dans les gammes précitées.

Dans le cas de la figure 3, une seule résistance 29 est placée en série avec l'électrode métallique 19, mais dans ce cas, trois circuits d'entrée sélectifs doivent être connectés aux bornes de cette résistance pour séparer les informations relatives à chacune des fréquences.

La figure 4 est une application multi-voies du schéma de la figure 3. Dans ce cas, sept électrodes métalliques 3oa à 30g sont connectées en série avec sept résistances 31a à 31g. Un circuit de commutation permet de relever successivement les tensions aux bornes des résistances 31a à 31g pour chacune des fréquences des générateurs 20, 21 et 22 et d'en déduire ainsi, d'une part les impédances, et d'autre part, les angles de déphasage pour chacune des voies de mesure, c'est-à-dire pour chacun des ensembles de ferraillage situés en-dessous de chaque électrode.

Les résultats obtenus comme décrits précédemment, peuvent être complétés comme représentés aux figures 8 ou 9.

Dans ce cas, on interrompt les signaux appliqués et on mesure les tensions intrinsèques d'une part entre le ferraillage et la surface du béton $(V_1)$ et d'autre part entre le ferraillage et son environnement direct $(V_2)$.

Pour cela, on utilise dans la figure 8 deux électrodes de références 32 et 33, par exemple des électrodes $Cu/SO_4Cu$, l'électrode 32 étant placée à la surface du béton immédiatement à proximité du ferraillage 2 et l'électrode 33 étant disposée à la surface du béton à côté d'une électrode métallique 34 reliée au ferraillage.

La tension $V_1$ est mesurée entre l'électrode de référence 32 et le ferraillage, et la tension $V_2$ est mesurée entre l'électrode de référence 33 et l'électrode métallique 34.

Dans le cas de la figure 9, une seule électrode de référence 35 est utilisée et un commutateur 36 est disposé entre le ferraillage 2 et l'électrode métallique 34. On constate immédiatement que la tension $V_x$ mesurée entre l'électrode de référence 35 et le ferraillage est égale à $V_1$ lorsque le commutateur 36 est ouvert et à $V_2$ lorsque le commutateur 36 est fermé.

Dans les deux cas, la comparaison de $V_1$ et de $V_2$ donne immédiatement le sens du courant entrant ou sortant du ferraillage.

Si $| -V_2 | > | -V_1 |$ le courant est sortant et il y a corrosion. Si au contraire, $| -V_2| < | -V_1 |$ alors le courant entre dans le ferraillage et celui-ci ne se corrode pas.

Diverses variantes et modifications peuvent bien entendu être apportées à la description qui précède sans sortir pour autant ni du cadre, ni de l'esprit de l'invention.

**Revendications**

1. Procédé de diagnostic de la corrosion des ferraillages d'une structure en béton armé, caractérisé par le fait que l'on applique au moins un signal électrique entre ledit ferraillage et une électrode placée à la surface du béton au voisinage dudit ferraillage, que l'on relève les caractéristiques de la tension et de l' intensité dudit signal, et que l'on déduit desdites caractéristiques au moins un paramètre représentatif de l'état de corrosion du ferraillage.

2. Procédé selon la revendication 1, caractérisé par le fait que l'un au moins desdits signaux électriques est un signal alternatif de fréquence prédéterminée, que l'on détermine l'angle de déphasage entre son intensité et sa tension, et que l'on déduit l'état de corrosion du ferraillage dudit angle de déphasage.

3. Procédé selon l'une quelconque des revendications 1 et 2, caractérisé par le fait que l'un au moins desdits signaux électriques est un signal alternatif dans une gamme de fréquences relativement basse, et que l'on déduit la résistance de polarisation du ferraillage du rapport entre les amplitudes de sa tension et de son intensité.

4. Procédé selon la revendication 3, caractérisé par le fait que l'on applique en outre au moins un deuxième signal alternatif dans une gamme de fréquences relativement haute, et que l'on déduit la résistance du béton compris entre le ferraillage et l'électrode du rapport entre les amplitudes de la tension et de l'intensité dudit deuxième signal alternatif.

5. Procédé selon l'une quelconque des revendications 1 à 4, caractérisé par le fait que l'on applique au moins deux signaux électriques, et que l'on déduit la résistance de polarisation du ferraillage du gradient amplitude de l'intensité/amplitude de la tension.

6. Procédé selon l'une quelconque des revendications 1 à 5, caractérisé par le fait que l'on interrompt ledit signal électrique, que l'on mesure alors d'une part la tension du ferraillage par rapport à la surface du béton et d'autre part la tension du ferraillage par rapport à son environnement direct, et que l'on déduit l'état de corrosion du ferraillage du signe de la différence entre ces deux tensions.

7. Procédé de diagnostic de la corrosion des ferraillages d'une structure en béton armé, caractérisé par le fait que l'on met en oeuvre au moins deux des procédés selon l'une quelconque des revendications 2 à 6.

*Fig.1*

*Fig.2*

*Fig.3*

*Fig.4*

*Fig. 5*

*Fig. 6*

*Fig. 7*

*Fig. 8*

*Fig. 9*

Office européen
des brevets

**RAPPORT DE RECHERCHE EUROPEENNE**

Numero de la demande

EP 89 40 2114

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | GB-A-2 200 459 (NIPPON STEEL CO.) * En entier * --- | 1,2 | G 01 N 17/00 |
| Y | GB-A-2 157 441 (TAYLOR WOODROW) * Page de garde * --- | 1-4,7 | |
| Y | FR-A-2 527 772 (ELF) * En entier * --- | 1-4,7 | |
| A | GB-A-2 025 056 (KASAHARA) * Page de garde * ----- | 1,7 | |

DOMAINES TECHNIQUES
RECHERCHES (Int. Cl.5)

G 01 N
C 23 F

Le présent rapport a été établi pour toutes les revendications

| Lieu de la recherche | Date d'achèvement de la recherche | Examinateur |
|---|---|---|
| LA HAYE | 02-10-1989 | KOUZELIS D. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant

EPO FORM 1503 03.82 (P0402)